# EUROPÄISCHE PATENTSCHRIFT

(11) **EP 0 868 193 B1**
(45) Veröffentlichungstag und Bekanntmachung des Hinweises auf die Patenterteilung: **09.02.2000**
(21) Anmeldenummer: 96943940.5
(22) Anmeldetag: 12.12.1996
(51) Int. Cl.: A61K 35/78, F26B 7/00, C11B 9/02

(54) **VERFAHREN ZUR SCHONENDEN KEIMREDUZIERUNG VON PHARMAZEUTISCHEN ZUBEREITUNGEN**
PROCESS FOR GENTLY STERILISING PHARMACEUTICAL COMPOSITIONS
PROCEDE POUR STERILISER AVEC MENAGEMENT DES COMPOSITIONS PHARMACEUTIQUES

(30) Priorität: 21.12.1995 DE 19547973
(43) Veröffentlichungstag der Anmeldung: 07.10.1998
(73) Patentinhaber: Plantamed Arzneimittel GmbH, 92318 Neumarkt (DE)
(72) Erfinder: JOSEPH, Heinz, Walter, D-71522 Backnang (DE)
(74) Vertreter: Schwabe - Sandmair - Marx
(86) Internationale Anmeldenummer: EP9605578
(87) Internationale Veröffentlichungsnummer: WO9723232

(56) Entgegenhaltungen:
- EP-A- 0 435 302
- EP-A- 0 753 306
- DATABASE WPI Section Ch, Week 9624 Derwent Publications Ltd., London, GB; Class B04, AN 96-233025 XP002029962 & HU 210 015 B (SZARVADY B) , 28.Juli 1995

## Beschreibung

Pharmazeutische Zubereitungen wie beispielsweise geschnittene oder pulverisierte Drogen und Lebensmittel, z.B. geschnittene oder gemahlene Gewürzdrogen, dürfen in Deutschland oder auch in anderen Ländern nicht mit an sich hochwirksamen, dem besten Stand der Technik entsprechenden Methoden entkeimt werden. Dazu zählt insbesondere die Behandlung des Drogenmaterials mit keimreduzierenden Methoden, die sich ionisierender Strahlen, z.B. des Kobalt 60 oder Linearbeschleunigern bedienen, sowie des chemischen Verfahrens der Begasung mit Ethylenoxid. Obschon diese Bestrahlungsverfahren zur Keimreduzierung in pflanzlichen Drogen oder Lebensmitteln zu den wirksamsten Methoden gehören, bestehen jedoch aufgrund starker Bedenken nationale Verbote durch den Gesetzgeber, solche Verfahren zum Einsatz zu bringen. Diese Problematik steht mit der Gefahr der Bildung toxischer Substanzen im Zusammenhang.

Auch sogenannte schonende Verfahren, wie z.B. das hydrothermische Verfahren sowie die Behandlung mit gespanntem Wasserdampf können negative Veränderungen an dem zu entkeimenden Material nach sich ziehen. Dies trifft insbesondere auf Drogenmaterial zu, das empfindliche ätherische Öle enthält, die auf diese Weise sowohl pharmakologisch als auch geschmacklich verändert werden können. Mit einer derartigen hydrothermischen Methode ist auch ein Verlust an dem Gehalt an Inhaltsstoffen verbunden. Alle diese Nachteile sind daher aus verständlichen Gründen nicht wünschenswert. Die genannten Nachteile sind aber auch aus ökologischen Gründen zu vermeiden, da aufgrund der Wirkstoffverluste ein Mehrverbrauch an Pflanzenmaterial resultiert.

Ein besonders schwerwiegender Nachteil der hydrothermischen Verfahren ist die Tatsache, daß diese bei hohen Temperaturen arbeiten, z.B. einer Sattdampftemperatur zwischen 110 und 160°C. Daraus wird ersichtlich, daß insbesondere Drogen mit ätherischen Ölen zwar entkeimt werden, aber bedauerlicherweise ihre Ausgangscharakteristik verlieren. Neben der physikalischen Verringerung von Substanzen im Ausgangsmaterial ergeben sich chemische Veränderungen, durch die weitere Inhaltsstoffe des Drogenmaterials irreversibel umgewandelt oder abgebaut werden. Sowohl durch die hohen Temperaturen bei diesen hydrothermischen Entkeimungsverfahren als auch durch die Bestrahlung entstehen Sauerstoffradikale, wodurch der Flavonoid- und Polyphenolgehalt des Drogenmaterials irreversibel verändert werden. Im Falle der Begasung mit Ethylenoxid entstehen cancerogene Zwischenstufen der Pflanzeninhaltsstoffe, die in jedem Falle zu vermeiden sind.

Bei diesen bekannten Verfahren hat sich auch herausgestellt, daß aufgrund der Bildung von Dauersporen der Mikroorganismen die weitgehende Reduzierung beispielsweise von Schimmelpilzen äußerst schwierig ist. Aufgrund ihrer Eigenschaften neigen diese Keime bzw. deren Sporen dazu, nach ihrem Verbleib unter teilweise scharfen Sterilisationsbedingungen sich beim Stehenlassen weder zu vermehren.

Die Grenzen der Keimbelastung von beispielsweise pflanzlichen Drogen sind für Arzneimittel mit einem Gehalt an derartigen Materialien im DAB 10(deutsches Arzneibuch 10. Ausgabe) festgelegt. Häufig müssen daher Drogen, obwohl sie an sich chemisch oder pharmazeutisch gesehen verwertbar wären, verworfen werden, weil sie den strengen Anforderungen der Arzneibücher, beispielsweise des DAB 10, nicht genügen.

Für pflanzliche Arzneimittelzubereitungen schreibt das DAB 10 folgende maximale Keimwerte vor: für Aerobier 10⁵, für Pilze und Hefen 10³, für Enterobakterien 10³, für E. Coli 10¹, wobei keine Salmonellen enthalten sein dürfen. Bezüglich von pflanzlichen Drogen, beispielsweise Arzneitees, die vor Anwendung (Brühen) eine Keimzahlverminderung erfahren oder bezüglich äußerlich anzuwendender pflanzlicher Arzneimittelzubereitungen gelten im DAB 10 höhere maximal zulässige Keimzahlwerte. Als Höchstwerte sind für Aerobier in diesem Falle 10⁷, für Pilze und Hefen 10⁴, für Enterobakterien 10⁴, für E. Coli 10² und für Salmonellen 0 pro g Material festgelegt. Für das ab 1.1.1996 national gültige Europäische Arzneibuch gelten ganz ähnliche Werte, wobei dieses bei pflanzlichen Arzneimittelzubereitungen zur innerlichen Verabreichung sogar eine Höchstzahl von 10⁴ Pilzen zuläßt, wobei jedoch keine E. Coli- oder Salmonellenkeime vorhanden sein dürfen. Die Maximalkeimzahlempfehlungen des deutschen Fachausschusses für Arznei-, Gewürz- und Aromapflanzen der Erzeuger von Arznei- und Gewürzpflanzen lehnen sich an die Vorschriften des DAB 10 an, so daß die nämlichen maximalen Keimzahlwerte zu beachten sind. Das Schweizerische Arzneibuch (Pharmacopöa Helvetica VII) erlaubt einen maximalen Keimgehalt von Hefen mit 10⁴/g, wobei im Hinblick auf Schimmelpilze lediglich keine wahrnehmbare Verschimmelung zugelassen ist, bei E. Coli höchstens 10²/g erlaubt sind und Salmonellen nicht nachweisbar sein sollten.

Es hat sich herausgestellt, daß die Einhaltung der Arzneibuchvorschriften oder der Empfehlungen der Fachausschüsse äußerst schwierig ist, ohne dabei, wie bereits oben dargestellt, den Gehalt an Wirkstoffen zu vermindern oder deren chemische Struktur und pharmakologische Wirkung in unerwünschter Weise zu verändern.

Die Aufgabe der Erfindung besteht deshalb darin, ein Verfahren zu schaffen, das eine starke Reduzierung von Keimen unter gleichzeitigem Erhalt der Struktur und qualitativen und quantitativen Wirkung sowie Menge der Inhaltsstoffe ermöglicht.

In völlig überraschender Weise wurde nunmehr gefunden, daß diese Aufgabe unter Überwindung der Nachteile des Standes der Technik unter den nachstehend bezeichneten Betriebsbedingungen in dem im folgenden gekennzeichneten Vakuumtrocknungssystem gelöst wird. Ein derartiges Trocknungssystem ist unter der Bezeichnung INOX-MAURER-Vakuumtrockner bekannt. Entsprechende Systeme sind auch noch unter der Bezeichnung Riniker-MZA-Mehrzweckanlage oder INOX-GLATT(Maurer)-Vakuumtrockner bekannt. Diese Vakuum-Trocknungssysteme zeichnen sich dadurch aus, daß sie ein durch eine zylindrische Misch- und Trocknerkammer erstreckendes mehrschenkliges Rührwerk mit eigenem Antrieb aufweisen, wobei diese Systeme erforderlichenfalls jeweils mit einem Filter, einer Rückspülungseinrichtung, einem Lösungsmittelkondensator mit Nachkühler und Auffanggefäß, Rückkondensator und/oder einer Prozeß-, Steuer- und Regeleinheit versehen sind. Als handelsübliche Geräte seien beispielsweise IUT-INOX-Universaltrockner, z. B. die Type IUT 20 oder IUT 50 genannt. Die Druckverhältnisse bei der Durchführung des erfindungsgemäßen Entkeimungsverfahrens werden so eingestellt, daß beim Evaporieren des zur Entkeimung eingesetzten Wasser/Ethanolgemisches ein Druck von 1 bis 600 mbar, bevorzugterweise von 10 bis 200 mbar und in besonders bevorzugter Weise von 50 bis 150 mbar eingestellt wird.

Die geeigneten Konzentrationen an Ethanol in dem hydroalkoholischen Gemisch beträgt zwischen 10 und 96 %, in bevorzugter Weise von 30 bis 90 % bzw. 40 bis 80 %. Ein weiterer bevorzugter Bereich beträgt 20 bis 75 %, wobei eine 70 %ige Konzentration ganz besonders bevorzugt wird. Das Mengenverhältnis von zu entkeimender, ggf. gepulverter Droge zu dem Ethanol/Wassergemisch beträgt 100 : 50 bis 50 : 100.

Die bei der Trocknung zu wählenden Temperaturen liegen bei dem Heizmantel zwischen 25°C und 90°C, wobei der Bereich von 65°C bis 75°C bevorzugt ist. Die Produkttemperatur wird dabei so eingeregelt, daß sie 10 bis 60°C, in bevorzugter Weise 20 bis 40°C und ganz besonders bevorzugt 25 bis 35°C beträgt.

Es wird bei der Entkeimung so vorgegangen, daß geschnittene oder gemahlene Drogen, ggf. zur Weiterverarbeitung zu Arzneimittelzubereitungen, mit einem Gemisch aus Ethanol und Wasser versetzt werden, wobei ein Gemisch aus 80 % Ethanol und der Rest Wasser bzw. 70% Ethanol und der Rest Wasser besonders bevorzugt wird, und nach der Einwirkung über mehrere Tage, beispielsweise von 2 bis 15 Tagen, im Anschluß daran schonend mit dem vorstehend beschriebenen Trocknungssystem unter Einhaltung der obenstehend beschriebenen Parameter behandelt werden.

Die besonders bevorzugten Mengenverhältnisse von Droge zu dem Ethanol/Wasserge-misch betragen 100 : 90 bis 10 : 90, insbesondere 50 : 50.

Alle Prozent- und Verhältnisangaben beziehen sich auf die jeweiligen Werte als Gewicht.

Je nach Bedarf und Konsistenz des eingebrachten zu entkeimenden Gutes wird das Rührwerk zugeschaltet.

Zum Aufzeigen der unerwarteten Vorteile des erfindungsgemäßen Entkeimungsverfahrens sind in der folgenden Tabelle eine Reihe von Vergleichswerten auf der Grundlage häufig verwendeter Pflanzen Drogen aufgelistet. Um die Überlegenheit des erfindungsgemäßen Entkeimungsverfahrens zu verdeutlichen, wurde eine vergleichende Entkeimung auf der Grundlage eines Trocknungsverfahrens durchgeführt, wobei das Abtrocknen der zu behandelnden Droge bzw. die Entfernung des Alkohol/Wassergemisches zum Vergleich unter den Normaldruckbedingungen gemäß Stand der Technik erfolgte. Die Produkttemperatur während des Trocknens nach dem Stande der Technik lag ebenso wie beim erfindungsgemäßen Entkeimen zwischen 25 und 35°C; dies geschah in einer üblichen Trocknungskammer.
- Beispiel 1:: Flos Sambuci
- Beispiel 2:: Flos Primulae
- Beispiel 3:: Herba Rumicis

Die jeweiligen Keimausgangswerte werden in der folgenden Tabelle im Vergleich zu den Keimwerten nach der erfindungsgemäßen Entkeimung und der Sterilisation gemäß Stand der Technik anhand der vorstehenden Beispiele einander gegenübergestellt.

| Vergleichende Entkeimungswerte | | | | | | | | | |
|---|---|---|---|---|---|---|---|---|---|
| Beispiel | 1) Flos Sambuci | | | 2) Flos Primulae | | | 3) Herba Rumicis | | |
| Keimart | vor Entk. | nach erfind. Entk. | nach Entk. St.d.T. | vor Entk. | nach erfind. Entk. | Entk. gemäß St. d.T. | vor Entk. | nach erfind. Entk. | nach Entk. St.d.T. |
| Aerob.+ Anaer. | 10⁵ | 10⁵ | 10⁵ | 10⁵ | 10⁴ | 10⁴ | 10⁸ | 10⁵ | 10⁴ |
| Hefepilze | 10³ | 10² | 10³ | 10² | 10² | 10³ | 10⁴ | 10³ | 10³ |
| Schimmelpilze | 10³ | 10² | 10⁴ | 10³ | 10³ | 10⁵ | 10⁴ | 10³ | 10⁵ |
| Enterobak. | 10³ | 10³ | 10² | 10⁴ | 10³ | 10³ | 10⁵ | 10² | 10² |
| E. Coli | 10² | 10¹ | 10¹ | 10² | 10¹ | 10¹ | 10² | 10¹ | 10¹ |
| Salmon. | o | o | o | o | o | o | o | o | o |

Die in der vorstehenden Tabelle dargestellten Entkeimungswerte machen in völlig überraschender Weise deutlich,daß es mit dem erfindungsgemäßen Entkeimungsverfahren möglich ist, die Arzneibuchvorschriften einzuhalten. Dies gilt insbesondere für die Einhaltung der Maximalwerte für Schimmelpilze, die aufgrund der Bildung lebertoxischer Aflatoxine in Lebensmittel- und Arzneimittelzubereitungen höchst unerwünscht sind. Was den Maximalgehalt an E. Coli-Keimen anlangt, sei darauf hingewiesen, daß die Anzahl von 10¹ eine Aufrundung auf die Zahl 10 bedeutet, wobei die tatsächlich aufgefundenen Werte zwischen 1 und 9 lagen, so daß solche Keime in Dosiseinheiten von Arzneimitteln, die aus den entkeimten Proben hergestellt werden, unterhalb der Nachweisgrenze vorhanden wären.

Die Vergleichsversuchsergebnisse belegen, daß mit dem Entkeimungsverfahren gemäß Stand der Technik die Anzahl, insbesondere der Schimmelpilze, sogar in einem Ausmaße zunimmmt, daß die so entkeimten Drogen den Arzneibuchvorschriften nicht genügen und deshalb zu verwerfen sind.

## Patentansprüche

1. Verfahren zum Entkeimen von insbesondere pflanzlichen Drogen, die ggf. vermahlen sind, dadurch **gekennzeichnet,** daß die Entkeimung in einem Vakuumtrocknungssystem erfolgt, das ein sich durch die zylindrische Misch- und Trocknerkammer erstreckendes mehrschenkliges Rührwerk mit eigenem Antrieb aufweist, sowie erforderlichenfalls mit einem Brüdenfilter, einer Rückspülungseinrichtung, einem Lösungsmittelkondensator mit Nachkühler und Auffanggefäß, Rückkondensator und ggf. mit einer Prozeß- Steuer- und Regeleinheit versehen ist, wobei das zu entkeimende Gut in dem mit einem sich über die Gesamttiefe der Trockner- und Mischkammer erstreckenden Zerhacker mit durch einen kammförmigen Stator hindurch rotierenden Messer ausgestatteten Trockner bei einer Heizmanteltemperatur von 25 bis 90°C, bevorzugt von 65 bis 75°C und einer
Produkttemperatur von 10 bis 60°C, bevorzugt von 20 bis 40°C und ganz besonders bevorzugt bei 25 bis 35°C, mit einem
Ethanolwassergemisch mit einem Gehalt an 10 bis 90 Gewichtsprozent, bevorzugt zwischen 20 und 80 Gewichtsprozent, in bevorzugterer Weise zwischen 40 und 85 % und ganz besonders bevorzugt mit 70 Gewichtsprozent Ethanol
bei einem Druck von 1 bis 600 mbar, bevorzugt von 10 bis 200 mbar und ganz besonders bevorzugt zwischen 50 und 150 mbar behandelt wird, wobei das
Mengenverhältnis in Gewicht von dem Alkohol/Wassergemisch : Droge von 100 : 90 bzw. 50 : 100 bis 10 : 90 bzw. 100 : 50, und ganz besonders bevorzugt 50 : 50 Gewichtsteile beträgt.

2. Verfahren nach Anspruch 1, dadurch gekennzeichnet, daß die zu entkeimende Droge bzw. zu entkeimenden Drogen aus Arten von Althaea, Rosmarinus, Juglans, Millefolium, Centaurium, Rosmarinum, Gentiana, Primula, Rumex, Sambuco und Verbena in Form der jeweiligen Blüten, Wurzeln bzw. Rhizomen oder Knollen, Blättern, oder anderen Pflanzenteilen ausgewählt sind.

3. Verfahren nach einem der Ansprüche 1 oder 2, dadurch gekennzeichnet, daß das zu entkeimende Drogenmaterial Flos Sambuci, Flos Primulae oder Herba Rumicis darstellt.

4. Verfahren nach den Ansprüchen 1 - 3, dadurch gekennzeichnet, daß das pflanzliche Drogenmaterial aus Radix Althaeae, Folium Rosmarini, Folium Juglandis, Herba Millefolii, Herba Centaurii, oder Radix Gentianae ausgewählt ist.

## Claims

1. Method for the sterilisation of plant-based drugs, in particular, which may have been ground, characterised in that the sterilisation takes place in a vacuum drying system with a multi-knuckled stirrer with its own drive which extends into the cylindrical mixing and drying chamber, as well as with a vapour filter if required, a backwash device, a solvent condenser with an after cooler and collection vessel, a return condenser and a process control and regulating unit as required, whereby the material to be sterilised is treated in the dryer fitted out with a chopper which extends to the total depth of the drying and mixing chamber with a knife rotating through a comb-shaped stator at a heated chamber wall temperature of between 25°C and 90°C, whereby 65°C to 75°C is the preferred range, and a product temperature of between 10°C and 60°C, whereby 20°C to 40°C is the preferred range and a particularly preferred range is 25°C to 35°C, with an ethanol/water mixture with 10 to 90 percent by weight of ethanol, whereby 20 to 80 percent is the preferred range, 40 to 85 percent is a particularly preferred range and the very particularly preferred content is 70 percent by weight of ethanol at a pressure of between 1 and 600 mbar, whereby between 10 to 200 mbar is the preferred range and between 50 and 150 mbar is a particularly preferred range, whereby the weight relationship of the alcohol/water mixture : drug combination ranges from 100 : 90 or 50 :100 to 10:90 or 100:50 and in particularly 50:50 by weight.

2. Method according to Claim 1, characterised in that the drug to be sterilised or drugs to be sterilised are chosen from plants of the genus Althae, Rosemarinus, Juglans, Millefolium, Centaurium, Rosemary, Gentiana, Primula, Rumex, Sambucus and Verbena in the form of each of the plant's petals, roots, rhizomes or bulbs, leaves or any other part of the plant.

3. Method according to Claims 1 or 2, characterised in that the drug material to be sterilised is Flores Sambuci, Flores Primulae or Herba Rumicis.

4. Method according to Claims 1 to 3, characterised in that the plant-based drug material is chosen from Radix Althaeae, Folium Rosmarini, Folium Juglandis, Herba Millefolii, Herba Centaurii or Radix Gentianae.

## Revendications

1. Procédé de stérilisation douce en particulier de substances pharmaceutiques végétales, qui sont éventuellement moulues, caractérisé en ce que la stérilisation s'effectue dans un système de déshydratation sous vide qui comporte un agitateur à plusieurs bras qui s'étend à travers la chambre cylindrique de malaxage et de séchage et qui dispose de son propre dispositif d'entraînement et qui au besoin est pourvu d'un filtre à buées, d'un dispositif de lavage à contre-courant, d'un condenseur de solvant avec réfrigérant secondaire et récipient de collecte, d'un condenseur de retour et éventuellement d'une unité de commande et de régulation du processus, le produit à stériliser étant traité dans le séchoir équipé d'un hachoir qui s'étend sur toute la profondeur de la chambre de séchage et de malaxage et qui présente une lame tournant à travers un stator en forme de peigne, à une température de l'enveloppe de chauffage de 25 à 90°C, de préférence de 65 à 75°C et
à une température du produit de 10 à 60°C, de préférence de 20 à 40°C et de manière tout particulièrement préférée de 25 à 35°C, avec un
mélange d'éthanol et d'eau d'une teneur de 10 à 90 pour cent en poids, de préférence entre 20 et 80 pour cent en poids, de manière plus préférée entre 40 et 85 % et de manière tout particulièrement préférée de 70 pour cent en poids d'éthanol
à une pression de 1 à 600 mbar, de préférence de 10 à 200 mbar et de manière tout particulièrement préférée entre 50 et 150 mbar,
le rapport en poids des quantités du mélange alcool/eau : substance pharmaceutique variant entre 100 : 90 respectivement 50 : 100 et 10 : 90, respectivement 100 : 50, et étant de manière tout particulièrement préférée de 50 : 50.

2. Procédé selon la revendication 1, caractérisé en ce que la substance ou les substances pharmaceutiques à stériliser sont sélectionnées parmi des variétés d'althaea, rosmarinus, juglans, millefolium, centaurium, rosmarinum, gentiana, primula, rumex, sambuco et vervena sous la forme des fleurs, racines respectivement rhizomes ou tubercules, feuilles ou d'autres parties des plantes.

3. Procédé selon l'une des revendications 1 ou 2, caractérisé en ce que la substance pharmaceutique à stériliser constitue flos sambuci, flos primulae ou herba rumicis.

4. Procédé selon les revendications 1 à 3, caractérisé en ce que la matière de la substance végétale est sélectionnée parmi le radix althaeae, folium rosmarini, folium juglandis, herba millefolii, herba centaurii ou radix gentianae.
